# EUROPÄISCHE PATENTSCHRIFT

(11) **EP 2 088 186 B1**
(45) Veröffentlichungstag und Bekanntmachung des Hinweises auf die Patenterteilung: **31.07.2013**
(21) Anmeldenummer: 09154885.9
(22) Anmeldetag: 24.11.2004
(51) Int. Cl.: C11D 3/37, C11D 17/00, C11D 3/22, A61K 8/02, A61Q 5/02, A61Q 19/10

(54) **Mehrkomponenten-Thin-To-Thick-System**
Multi-component thin-to-thick system
Système mince à épais à plusieurs composants

(30) Priorität: 04.03.2004 DE 102004010485; 13.12.2003 DE 10358536
(43) Veröffentlichungstag der Anmeldung: 12.08.2009
(62) Teilanmeldung aus: 04798063.6
(73) Patentinhaber: Henkel AG & Co. KGaA, 40589 Düsselsdorf (DE)
(72) Erfinder: BEDRUNKA, Danuta, 41541, Dormagen (DE); HLOUCHA, Matthias, 50677, Köln (DE); HOFMANN, Rainer, 50389, Wesseling (DE); HATTEMER, Erik, 40597, Düsseldorf (DE); RYBINSKI VON, Wolfgang, 40593, Düsseldorf (DE); DAHLMANN, Doris, 40589, Düsseldorf (DE)

(56) Entgegenhaltungen:
- EP-A- 0 875 557
- EP-A- 0 982 021
- DE-A1- 19 752 163
- US-A- 5 004 557
- US-A- 5 362 415
- US-A- 5 652 208
- ILIOPOULOS, WANG, AUDEBERT: LANGMUIR, Nr. 7, 1991, Seiten 617-619, XP008042857

## Beschreibung

Die vorliegende Erfindung betrifft ein Mehrkomponenten-System, das beim Vermischen eine hochviskose Flüssigkeit ausbildet, ein Verfahren zur Herstellung einer derartigen hochviskosen Flüssigkeit sowie die Verwendung dieses Mehrkomponenten-Systems, insbesondere als kosmetisches Mittel oder als Wasch- und/oder Spül- und/oder Reinigungsmittel.

In der Technik besteht allgemein ein Bedarf nach Reinigungsmitteln mit hoher Viskosität, um eine verlängerte Kontaktzeit mit der Oberfläche zu realisieren und dadurch den Reinigungseffekt zu verbessern. Ein Problem bei hochviskosen Flüssigkeiten besteht jedoch darin, dass diese in der Praxis nur schlecht zu handhaben sind. So ist es beispielsweise nicht oder nur schwer möglich hochviskose Reinigungsmittel zu dosieren oder unter Verwendung einer Sprühvorrichtung zu applizieren.

Eine Lösung dieses Problems würde etwa darin bestehen, das hochviskose Reinigungsmittel am Applikationsort selbst zu erzeugen. In diesem Sinne wurden Reinigungsmittel entwickelt, die eine niedrige Viskosität besitzen und erst beim Verdünnen mit Wasser eine höhere Viskosität annehmen. So beschreibt etwa EP0595590 ein Konzentrat, das Aminoxide, ein anionisches Alkyl-Detergens, ein hydrophob-modifiziertes Polymer und einen Verdünner enthält. Bei Zugabe von 96 bis 98 Teilen Wasser bildet dieses Konzentrat eine hochviskose Flüssigkeit aus.

Aufgabe der vorliegenden Erfindung war es daher Alternativen zu finden, die es erlauben, am Applikationsort selbst eine hochviskose Flüssigkeit bereitzustellen. Aufgabe der vorliegenden Erfindung war es hierbei insbesondere, eine besser handhabbare Alternative zu den im Stand der Technik beschriebenen Systemen zur Verfügung zu stellen.

Ferner war es Aufgabe der vorliegenden Erfindung, das Auslaufverhalten des Gemisches im Vergleich zum Stand der Technik zu verbessern und/oder eine bessere Verdickung als bei herkömmlichen Verdickersystemen zu ermöglichen.

Die Veröffentlichungen von Iliopoulos et al. (1991) Langmuir 7, 617-619 und Panmai et al. (1999) Colloids and Surfaces A 147, 3-15 beschreiben Flüssigkeiten, die hydrophob modifizierte Polymere enthalten und bei Zugabe von grenzflächenaktiven Substanzen ein Viskositätsmaximum durchlaufen.

Es wurde nun überraschenderweise gefunden, dass ausgehend von mindestens zwei definierten niedrig-viskosen Flüssigkeiten, von denen mindestens eine ein hydrophob modifiziertes Polymer enthält und von denen mindestens eine andere eine Mischung aus einem hydrophob modifizierten Polymer und Detergentien enthält, beim Vermischen dieser in geeignetem Verhältnis, eine hochviskose Flüssigkeit entsteht, insbesondere eine solche, die den Anforderungen eines Reinigungsmittels mit verlängerter Kontaktzeit in vorteilhafter Weise gerecht wird.

Auf diese Weise wird eine erhöhte Produkthaftung an Oberflächen und dadurch eine längere Einwirkzeit ermöglicht, was neben dem erhöhten Reinigungseffekt auch eine Visualisierung für den Verbraucher mit sich bringt.

Gegenstand der vorliegenden Erfindung ist daher ein Verfahren zur Herstellung einer hochviskosen Flüssigkeit, dadurch gekennzeichnet, dass mindestens zwei, vorzugsweise niedrigviskose, Flüssigkeiten miteinander vermischt werden, wobei die erste der mindestens zwei Flüssigkeiten mindestens ein hydrophob-modifiziertes Polymer enthält und die zweite der mindestens zwei Flüssigkeiten mindestens ein hydrophob-modifiziertes Polymer und mindestens ein Tensid in einer Menge zwischen 0,05 und 20 Gew.% enthält, wobei die Menge an hydrophob modifiziertem Polymer in den beiden Flüssigkeiten bis zu 5 Gew.-% beträgt, dass die erste und zweite Flüssigkeit im Verhältnis 1:2 bis 100:1 miteinander gemischt werden und dass es sich bei dem hydrophob modifizierten Polymer um ein hydrophob modifiziertes Polysaccharid handelt.

Gegenstand der vorliegenden Erfindung ist daher des weiteren ein Mehrkomponenten-System, vorzugsweise ein Zwei-Komponenten-System, umfassend
a) eine erste wässrige Flüssigkeit, die mindestens ein hydrophob-modifiziertes Polymer enthält,
b) eine zweite wässrige Flüssigkeit, die mindestens ein hydrophob modifiziertes Polymer und mindestens ein Tensid in einer Menge zwischen 0,05 und 20 Gew.-% enthält,
wobei es sich bei dem hydrophob modifzierten Polymer um ein hydrophob modifiziertes Polysaccharid handelt, das Verhältnis zwischen erster und zweiter Flüssigkeit zwischen 1:2 und 100:1 liegt und die Menge an hydrophob modifiziertem Polymer in beiden Flüssigkeiten bis zu 5 Gew.-% beträgt.

Außer der Verwendung eines Zwei-Komponenten-Systems ist es erfindungsgemäß auch möglich, ein System zu verwenden, das mehr als zwei Komponenten umfasst. In einer bevorzugten Ausführungsform handelt es sich jedoch bei dem Mehrkomponentensystem um ein Zweikomponenten-System. Zur Anwendung des Mehrkomponenten-Systems werden die Komponenten in einem geeigneten Verhältnis miteinander gemischt, wobei bei Verwendung eines Zwei-Komponenten-Systems das Verhältnis zwischen erster und zweiter wässriger Flüssigkeit zwischen 1:2 und 100:1 liegt.

Erfindungsgemäß beträgt die Menge an hydrophob-modifiziertem Polymer in den beiden Flüssigkeiten bis zu 5 Gew.-%, besonders bevorzugt liegt sie zwischen 0,1 und 2 Gew.-%.

Die erste wässrige Flüssigkeit des Mehrkomponenten-Systems muss kein Tensid enthalten und enthält in einer bevorzugten Ausführungsform auch kein Tensid. In einer weiteren bevorzugten Ausführungsform enthält die erste Flüssigkeit jedoch geringe Mengen an Tensid, vorzugsweise zwischen 0 und 0,4 Gew.-%, besonders bevorzugt zwischen 0 und 0,1 Gew.-%. Es kommt auch hierbei jedes denkbare Tensid oder Mischungen davon in Frage, insbesondere ein Tensid, das aus derselben Gruppe ausgewählt ist wie das Tensid der zweiten Flüssigkeit. In einer bevorzugten Ausführungsform sind in erster und zweiter Flüssigkeit die gleichen Tenside enthalten.

Die Menge an Tensid in der zweiten Flüssigkeit beträgt zwischen 0,05 und 20 %, besonders bevorzugt zwischen 0,1 und 12 %, vor allem zwischen 0,2 und 8 Gew.-%.

### Hydrophob modifiziertes Polymer

Bei dem hydrophob modifizierten Polymer handelt es sich um ein hydrophob modifiziertes Polysaccharid wie beispielsweise ein hydrophob modifiziertes CelluloseDerivat.

Das hydrophob modifizierte Polymer kann jede beliebige Struktur besitzen. So kann es beispielsweise linear, verzweigt, kammartig und/oder sternförmig sein.

Hydrophob modifiziert heißt erfindungsgemäß, dass hydrophobe Gruppen an das Polymer gebunden sind und zwar vorzugsweise an ein oder mehrere funktionelle Gruppen des Polymers. Die hydrophobe Gruppe kann über eine Alkylen-Gruppe, insbesondere C₁₋₆-Alkylen-Gruppe, vorzugsweise Methylen-Gruppe, an das Polymer-Rückgrat gebunden sein.

Der Modifizierungsgrad gibt hierbei das Verhältnis zwischen Anzahl der Monomereinheiten des Polymers und Anzahl der hydrophoben modifizierenden Gruppen an bzw. insbesondere, im Falle der Bindung an funktionelle Gruppen und vorausgesetzt, dass alle Monomere funktionelle Gruppe umfassen, die Anzahl der durch hydrophobe Gruppen modifizierten funktionellen Gruppen in Bezug auf die Gesamtzahl der funktionellen Gruppen.

Die hydrophobe Gruppe kann erfindungsgemäß beispielsweise eine Alkylgruppe, eine aromatische Gruppe oder eine Polyether-Gruppe umfassen und sie kann linear oder verzweigt sein. Bei der hydrophoben Gruppe handelt es sich hierbei erfindungsgemäß vorzugsweise um eine Alkylgruppe mit einer Länge zwischen C₈ und C₅₀, insbesondere zwischen C₈ und C₂₆, besonders bevorzugt zwischen C₁₂ und C₂₂, vor allem zwischen C₁₆ und C₂₀, insbesondere mit einer Länge von C₁₈, oder es handelt sich um ein Polyalkylenglykol, insbesondere ein Polyethylenglykol, ein Polypropylenglykol oder ein Copolymer aus Oxyethylen- und Oxypropylen-Einheiten, mit vorzugsweise 2 bis 50, besonders bevorzugt 2 bis 30 Wiederholungseinheiten, wobei die terminale Hydroxy-Gruppe des Polyalkylenglykols vorzugsweise verestert oder verethert ist, wobei die Ester-Bindung vorzugsweise ausgebildet ist mit einer Säure ausgewählt aus einer C₅₋₅₀-Carbonsäure, insbesondere einer C₈₋₂₆-Carbonsäure, besonders bevorzugt eine C₁₆₋₂₀-Carbonsäure, und wobei die Ether-Bindung vorzugsweise ausgebildet wird mit einem C₅₋₅₀-Alkohol, insbesondere einem C₈₋₂₆-Alkohol, besonders bevorzugt einem C₁₆₋₂₀-Alkohol.

Alle der zuvor genannten Kohlenwasserstoffreste sowie unabhängig davon das Rückgrat des Polymers können gegebenenfalls auch ein- oder mehrfach substituiert sein, insbesondere durch Reste ausgewählt aus Halogen, insbsondere Fluor, Chlor oder Brom, Hydroxy, Alkoxy, insbesondere C₁₋₆-Alkoxy, Amino, Alkylamino, insbesondere C₁₋₆-Alkylamino, Aryl, insbesondere C₆₋₁₀-Aryl Arylalkyl, insbesondere C₆₋₁₀-Aryl-C₁₋₆-alkyl, Carboxy, Carboxyester, insbesondere Carboxy-C₁₋₆-alkyl-ester, und cycloaliphatischen Resten.

Das hydrophob modifizierte Polymer oder dessen Derivat besitzt vorzugsweise ein Molekulargewicht kleiner als 100000 kDa, besonders bevorzugt zwischen 10 und 1000 kDa, insbesondere zwischen 50 und 600 kDa.

Der Modifizierungsgrad des hydrophob modifizierten Polymers liegt vorzugsweise zwischen 1 und 10 %, besonders bevorzugt zwischen 2 und 5 %, vor allem zwischen 2,5 und 3,5 %.

### Tenside

Bei dem erfindungsgemäß einsetzbaren Tensid handelt es sich vorzugsweise um ein Tensid ausgewählt aus der Gruppe bestehend aus anionischen Tensiden, amphoteren Tensiden und nichtionischen Tensiden oder Mischungen davon. Kationische Tenside können als zusätzliche Komponente in geringen Mengen vorhanden sein.

Als anionische Tenside werden beispielsweise solche vom Typ der Sulfonate und Sulfate eingesetzt. Als Tenside vom Sulfonat-Typ kommen dabei vorzugsweise C₉-₁₃-Alkylbenzolsulfonate, Olefinsulfonate, d.h. Gemische aus Alken- und Hydroxyalkansulfonaten sowie Disulfonaten. Geeignet sind auch Alkansulfonate. Ebenso sind auch die Ester von α-Sulfofettsäuren (Estersulfonate), z.B. die α-sulfonierten Methylester der hydrierten Kokos-, Palmkern- oder Talgfettsäuren geeignet.

Weitere geeignete anionische Tenside sind sulfierte Fettsäureglycerinester.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate, welche als Handelsprodukte der Shell Oil Company unter dem Namen DAN^{®} erhalten werden können, sind geeignete Aniontenside.

Auch die Schwefelsäuremonoester der mit 1 bis 6 Mol Ethylenoxid ethoxylierten geradkettigen oder verzweigten C₇₋₂₁-Alkohole (Alkylethersulfate), wie 2-Methylverzweigte C₉₋₁₁-Alkohole mit im Durchschnitt 3,5 Mol Ethylenoxid (EO) oder C₁₂-₁₈-Fettalkohole mit 1 bis 4 EO (Fettalkoholethersulfate), sind geeignet. Sie werden in Reinigungsmitteln aufgrund ihres hohen Schaumverhaltens nur in relativ geringen Mengen, beispielsweise in Mengen von 1 bis 5 Gew.-%, eingesetzt.

Weitere geeignete anionische Tenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈₋₁₈-Fettalkoholreste oder Mischungen aus diesen.

Die anionischen Tenside können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Als nichtionische Tenside können beispielsweise alkoxylierte, vorteilhafterweise ethoxylierte, insbesondere primäre Alkohole mit vorzugsweise 8 bis 18 C-Atomen und durchschnittlich 1 bis 20, insbesodere 1 bis 12, Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen.

Auch schwachschäumende nichtionische Tenside können eingesetzt werden, welche alternierende Ethylenoxid- und Alkylenoxideinheiten aufweisen. Unter diesen sind wiederum Tenside mit EO-AO-EO-AO-Blöcken bevorzugt, wobei jeweils eine bis zehn EO- bzw. AO-Gruppen aneinander gebunden sind, bevor ein Block aus den jeweils anderen Gruppen folgt.

Eine weitere Klasse einsetzbarer nichtionischer Tenside, die entweder als alleiniges nichtionisches Tensid oder in Kombination mit anderen nichtionischen Tensiden eingesetzt werden können, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Eine weitere Klasse von nichtionischen Tensiden, die erfindungsgemäß eingesetzt werden können, sind die Alkylpolyglycoside (APG). Einsetzbare Alkypolyglycoside haben die allgemeine Formel R-O(-G)_{z}, in der R für einen linearen oder verzweigten, insbesondere in 2-Stellung methylverzweigten, gesättigten oder ungesättigten, aliphatischen Rest mit 8 bis 22, vorzugsweise 8 bis 18 C-Atomen steht, G für einen glykosidisch gebundenen Rest eines Monosaccharids steht und z ein Wert zwischen 1 und 10 bedeutet.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete nichtionische Tenside sind Polyhydroxyfettsäureamide der Formel (II), 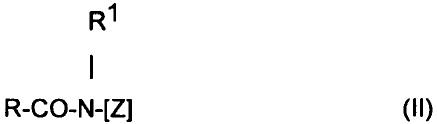 in der RCO für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht.

Geeignete amphotere bzw. zwitterionische Tenside, die erfindungsgemäß verwendet werden können, enthalten eine basische und eine saure Gruppe, die ein inneres Salz ausbilden. Bei der kationischen Gruppe handelt es sich hierbei insbesondere um eine quaternäre Ammoniumgruppe, es kann sich aber beispielsweise auch um eine Phosphonium-, Imidazolium- oder Sulfonium-Gruppe handeln. Bei der anionischen Gruppe handelt es sich insbesondere um eine Carboxylat- oder Sulfonat-Gruppe, es kann sich aber beispielsweise auch um eine Phosphonat- oder Sulfat-Gruppe handeln.

Typische Beispiele für amphotere bzw. zwitterionische Tenside sind Alkylbetaine, Alkylamidobetaine, Aminopropionate, Aminoglycinate, Imidazoliniumbetaine und Sulfobetaine. Im zuletzt genannten Fall ist statt der Carboxy- eine Sulfonatgruppe die anionische Gruppe. Besonders geeignet sind Bis(hydroxyethyl)sulfobetain und Cocoamidopropylsulfobetain. Es können jedoch allgemein Betaine und/oder Sulfobetaine eingesetzt werden, wie sie beispielsweise in US 2,082,275, US 2,702,279 oder US 2,255,082 beschrieben sind.

Besonders bevorzugt umfassen die eingesetzten Tenside mindestens ein Tensid ausgewählt aus der Gruppe bestehend aus Alkylsulfaten, Alkylethersulfaten, Alkylsulfonaten, Alkylarylsulfonaten, Alkylbenzolsulfonaten, Alkylarylethersulfonaten, Alkyletheroxylaten, Fettalkoholethersulfaten, LAS, APG, Betainen und Fettalkoholethoxylaten, insbesondere aus der Gruppe Dodecylsulfat, Dodecylethersulfat und Fettalkoholethoxylate umfassend einen C₁₂-C₁₈-Fettalkohol und im Mittel weniger als 20 Ethoxylat-Einheiten, vorzugsweise 6-7 Ethoxylat-Einheiten.

Die Anwendung des Mehrkomponentensystems, und insbesondere des Zwei-Komponenten-Systems, kann auf unterschiedliche Weise erfolgen. So können die mindestens zwei Komponenten in mindestens zwei getrennten Behältnissen, beispielsweise Flaschen, enthalten sein. Zur Herstellung der hochviskosen Flüssigkeit können die mindestens zwei Flüssigkeiten dann manuell in ein drittes Behältnis, beispielsweise in einen Eimer, dosiert und anschließend miteinander vermischt werden.

Alternativ kann aber etwa auch die Verwendung eines Mehrkammer-Systems, insbesondere eines Doppelkammer-Systems, beispielsweise eines Doppelkammer-Sprays oder einer Doppelkammer-Flasche, erfolgen. Eine manuelle Mischung der Flüssigkeiten ist hierbei nicht erforderlich, sondern die Mischung der Flüssigkeiten erfolgt automatisch bei Anwendung aufgrund der vorteilhaften Vorrichtung.

Die Applikation des Reinigungsmittels aus dem Behältnis, insbesondere aus dem Mehrkammersystem, auf den Applikationsort kann hierbei insbesondere auch unter Verwendung eines Sprühspenders erfolgen.

Bevorzugt ist der Sprühspender hierbei ein manuell aktivierter Sprühspender, insbesondere ausgewählt aus der Gruppe umfassend Aerosolsprühspender (Druckgasbehälter; auch u.a. als Spraydose bezeichnet), selbst Druck aufbauende Sprühspender, Pumpsprühspender und Triggersprühspender, insbesondere Pumpsprühspender und Triggersprühspender mit einem Behälter aus transparentem Polyethylen oder Polyethylenterephthalat. Sprühspender werden ausführlicher in der WO 96/04940 (Procter & Gamble) beschrieben. Triggersprühspender und Pumpzerstäuber besitzen gegenüber Druckgasbehältem den Vorteil, daß kein Treibmittel eingesetzt werden muß.

Zur Herstellung der hochviskosen Lösung werden die beiden Komponenten eines Zwei-Komponenten-Systems in einem Verhältnis von 1:2 bis 100:1, besonders bevorzugt in einem Verhältnis von 1:1 bis 50:1 miteinander gemischt. Das bevorzugte Mischungsverhältnis ist hierbei natürlich abhängig von der Zusammensetzung der beiden Komponenten.

Die Zusammensetzungen der beiden Komponenten des Zwei-Komponenten-Systems und deren Mischungsverhältnis werden in einer bevorzugten Ausführungsform so gewählt, dass die Viskosität des Gemisches bei einer Scherrate von 0,1 s⁻¹ etwa doppelt so hoch, dreifach so hoch, fünffach so hoch oder zehnfach so hoch ist wie die Viskosität mindestens einer, vorzugsweise beider, der beiden Komponenten.

In einer weiteren bevorzugten Ausführungsform des erfindungsgemäßen Mehrkomponentensystems, und insbesondere Zwei-Komponenten-Systems, werden die Zusammensetzungen und das Mischungsverhältnis der Komponenten so gewählt, dass die Viskosität der Mischung bei einer Scherrate von 0,1 s⁻¹ mehr als zehnfach, insbesondere mehr als 50fach, vor allem mehr als 100fach höher ist als die Viskosität mindestens einer, vorzugsweise beider bzw. aller, der eingesetzten Komponenten.

In bevorzugten Ausführungsformen besitzen die Komponenten des Mehrkomponentensystems unabhängig voneinander bei einer Scherrate von 0,1 s⁻¹ Viskositäten von kleiner als 100 und/oder kleiner als 200 Pas.

Die Viskositätsmessungen erfolgen mit Hilfe eines schubspannungskontrollierten Rotationsrheometers AR 1000-N (TA Instruments) mit einem Platte-Kegel-Meßsystem mit einem Durchmesser von 4 cm und einem Kegelwinkel von 1° in Fließversuchen im Schergeschwindigkeitsbereich von 0 bis 1000 s⁻¹ bei 20°C.

Der pH-Wert der Komponenten des Mehrkomponenten-Systems und ebenso der des resultierenden Gemischs liegt-vorzugsweise zwischen 5 und 14, wobei die einzelnen Komponenten des Mehrkomponenten-Systems und/oder das resultierende Gemisch unterschiedliche pH-Werte haben können.

In einer bevorzugten Ausführungsform enthält mindestens eine der Komponenten zusätzlich mindestens einen weiteren Bestandteil, wobei die Auswahl des weiteren Bestandteils sich nach dem Verwendungszweck richtet.

Als Verwendungszweck des erfindungsgemäßen Mehrkomponenten-Systems kommen beispielsweise die Verwendung als kosmetisches Mittel und/oder als Wasch- und/oder Reinigungsmittel in Frage.

Bei dem kosmetischen Mittel kann es sich hierbei insbesondere um ein Haarbehandlungs- und/oder Haarpflegemittel handeln, beispielsweise um ein Haarshampoo, eine Haarlotion, eine Haarkur oder ein Haarfärbemittel, oder um ein Körperpflegemittel, insbesondere Hautbehandlungsmittel, wie beispielsweise ein Deo, ein Sonnenöl oder eine Flüssigseife.

Bei dem Wasch- und/oder Reinigungsmittel kann es sich insbesondere um ein Textilbehandlungsmittel, insbesondere auch um ein Textilvorbehandlungsmittel, um ein Handwaschmittel, Handgeschirrspülmittel, insbesondere um ein Geschirrvorbehandlungsmittel, um ein Sanitärmittel, um ein Sterilisationsmittel und/oder um ein Desinfektionsmittel handeln.

Für die genannten Verwendungszwecke können entsprechend Inhaltsstoffe enthalten sein, wie sie dem Fachmann bekannt sind.

### Reiniger für harte Oberflächen

Als harte Oberflächen im Sinne dieser Erfindung kommen insbesondere alle im Haushalt üblichen Flächen aus Kunststoff, Glas, Keramik oder Metall in Frage, beispielsweise Küchenoberflächen, Herde, Badezimmerflächen, Fußbodenfliesen, Laminatböden oder Geschirr.

Bei Verwendung des Mehrkomponenten-Systems als Reiniger für harte Oberflächen können die Flüssigkeiten des Mehrkomponenten-Systems auch weitere Bestandteile enthalten, wie sie für ein Reiniger von harten Oberflächen dem Fachmann bekannt sind. Neben dem hydrophob modifizierten Polymer und den zuvor genannten Tensiden kann es sich hierbei insbesondere um Gerüststoffe (Builder), Säuren, Alkalien, Hydrotrope, Lösungsmittel, Verdickungsmittel, Abrasivstoffe, Enzyme sowie weitere Hilfs- und Zusatzstoffe wie Konservierungsmittel, Farbstoffe, Duftstoffe (Parfüme), Korrosionsinhibitoren oder Hautpflegemittel handeln. Soll das Reinigungsmittel versprüht werden, kann gegebenenfalls auch ein Treibmittel enthalten sein. Die verschiedenen Flüssigkeiten des mehrkomponentigen Reinigungsmittels können hierbei unabhängig voneinander eine oder mehrere der zuvor genannten und nachfolgend aufgezählten Komponenten in beliebiger Kombination und Konzentration enthalten.

Die Mittel können Builder in Mengen, bezogen auf die Zusammensetzung, von 0,1 bis 5 Gew.-% enthalten.

Säuren und/ oder Alkalien dienen einerseits als pH-Regulatoren, andererseits können die Säuren aber auch zur Entfernung von Kalkflecken von den zu reinigenden Oberflächen beitragen. Bei den erfindungsgemäß einsetzbaren Säuren kann es sich um anorganische Mineralsäuren, beispielsweise Salzsäure, und/oder um C₁₋₆- Mono-, Di-, Tri- oder Polycarbonsäuren oder -hydroxycarbonsäuren wie beispielsweise Ameisensäure, Essigsäure, Milchsäure, Citronensäure, Gluconsäure, Glutarsäure, Bernsteinsäure, Adipinsäure, Weinsäure oder auch Äpfelsäure sowie um weitere organische Säuren wie etwa Salicylsäure oder Amidosulfonsäure handeln. Besonders bevorzugt wird jedoch die Citronensäure eingesetzt; auch Mischungen aus mehreren Säuren können verwendet werden. Säuren können im erfindungsgemäßen Reinigungsmittel in Mengen von bis zu 6 Gew.-% enthalten sein, bezogen auf die Gesamtzusammensetzung.

Zu den gegebenenfalls einsetzbaren Basen zählen Alkanolamine, beispielsweise Mono- oder Diethanolamin, sowie Ammonium- oder Alkalimetallhydroxide, vor allem Natriumhydroxid. Das erfindungsgemäße Reinigungsmittel kann Basen in Mengen von bis zu 2,5 Gew.-% enthalten, bezogen auf die Gesamtzusammensetzung.

Die Komponenten des erfindungsgemäßen Mittels können weiterhin zur Viskositätsregulierung einen oder mehrere Verdicker enthalten. Als Verdickungsmittel geeignet sind dabei natürliche und synthetische Polymere sowie anorganische Verdickungsmittel. Zu den einsetzbaren Polymeren zählen Polysaccharide bzw. Heteropolysaccharide und andere organische natürliche Verdickungsmittel, darunter die Polysaccharidgummen wie Gummi arabicum, Agar, Alginate, Carrageene und ihre Salze, Guar, Guaran, Tragant, Gellan, Ramsan, Dextran oder Xanthan und ihre Derivate, z.B. propoxyliertes Guar, sowie ihre Mischungen, weiterhin auch Pektine, Polyosen, Johannisbrotbaumkernmehl, Stärke, Dextrine, Gelatine, Casein.

Neben den bisher genannten Verdickungsmitteln kann das erfindungsgemäße Reinigungsmittel Elektrolytsalze enthalten. Diese können ebenfalls zu einer Viskositätserhöhung beitragen. Elektrolytsalze im Sinne der vorliegenden Erfindung sind Salze, die in dem erfindungsgemäßen wäßrigen Mittel in ihre ionischen Bestandteile zerfallen. Bevorzugt sind die Salze, insbesondere Alkalimetall- und/oder Erdalkalimetallsalze, einer anorganischen Säure, vorzugsweise einer anorganischen Säure aus der Gruppe, umfassend die Halogenwasserstoffsäuren, Salpetersäure und Schwefelsäure, insbesondere die Chloride und Sulfate. Im Rahmen der erfindungsgemäßen Lehre kann ein Elektrolytsalz auch in Form seines korrespondierenden Säure/Base-Paares eingesetzt werden, beispielsweise Salzsäure und Natriumhydroxid anstatt Natriumchlorid.

In den Komponenten des erfindungsgemäßen Mittels sind die genannten oder andere organische und/oder anorganische Verdicker vorzugsweise nur in geringen Mengen enthalten. In einer bevorzugten Ausführungsform wird auf den Zusatz von Verdickern ganz verzichtet.

Die Komponenten des erfindungsgemäßen Mittels können vorteilhafterweise zusätzlich ein oder mehrere wasserlösliche organische Lösungsmittel enthalten, üblicherweise in einer Menge von bis zu 6 Gew.-%, bezogen auf die Gesamtzusammensetzung. Das Lösungsmittel wird im Rahmen der erfindungsgemäßen Lehre nach Bedarf insbesondere als Hydrotropikum, Viskositätsregulator und/oder Kältestabilisator eingesetzt. Es wirkt lösungsvermittelnd insbesondere für Tenside und Elektrolyt sowie Parfüm und Farbstoff und trägt so zu deren Einarbeitung bei, verhindert die Ausbildung flüssigkristalliner Phasen und hat Anteil an der Bildung klarer Produkte. Die Viskosität des erfindungsgemäßen Mittels verringert sich mit zunehmender Lösungsmittelmenge. Zuviel Lösungsmittel kann jedoch einen zu starken Viskositätsabfall bewirken. Schließlich sinkt mit zunehmender Lösungsmittelmenge der Kältetrübungs- und Klarpunkt des erfindungsgemäßen Mittels.

Die Komponenten des erfindungsgemäßen Reinigungsmittels können vorteilhafterweise zusätzlich ein oder mehrere Enzyme enthalten, vor allem ausgewählt aus der Gruppe bestehend aus Proteasen, Polysaccharidasen und Nukleasen. Bei der Polysaccharidase kann es sich insbesondere um eine β-Glucanase, Cellulase, Xylanase, Amylase, Dextranase, Glucosidase, Galactosidase, Pectinase, Chitinase, Lysozym und/oder Alginat-Lyase handeln. Bei der Protease kann es sich insbesondere um Subtilisin, Thermolysin, Pepsin, um eine Carboxypeptidase und/oder um eine saure Protease handeln. Bei den Nukleasen kann es sich um jede beliebige DNAse oder RNAse handeln.

Des weiteren kann das erfindungsgemäße Reinigungsmittel gegebenenfalls auch biozide Substanzen enthalten.

Als Lösungsvermittler können außer den zuvor beschriebenen Lösungsmitteln beispielsweise auch Alkanolamine sowie Alkylbenzolsulfonate mit 1 bis 3 Kohlenstoffatomen im Alkylrest, z.B. Xylol- oder Cumolsulfonat eingesetzt werden. Weitere einsetzbare Hydrotrope sind z.B. Octylsulfat oder Butylglucosid. Das erfindungsgemäße Mittel kann diese Hydrotrope in Mengen von, bezogen auf die Gesamtzusammensetzung, bis zu 4 Gew.-% enthalten.

In einer Ausführungsform können die erfindungsgemäßen Mittel Abrasivstoffe enthalten. Als Abrasivkomponente können hierbei feste wasserlösliche und wasserunlösliche, vorzugsweise anorganische Verbindungen und Gemische derselben dienen. Der Vorteil wasserlöslicher Abrasivkomponenten besteht in einer praktisch rückstandsfreien Abspülbarkeit des Mittels. Das erfindungsgemäße Reinigungsmittel kann Abrasivstoffe in Mengen von bis zu 2 Gew.% enthalten, bezogen auf die Gesamtzusammensetzung.

Neben den genannten Komponenten können die erfindungsgemäßen Mittel einen oder mehrere weitere Hilfs- und Zusatzstoffe enthalten, wie sie vor allem in Reinigungsmitteln für harte Oberflächen üblich sind. Hierzu zählen insbesondere UV-Stabilisatoren, Korrosionsinhibitoren, Reinigungsverstärker, Antistatika, Konservierungsmittel (z.B. 2-Brom-2-nitropropan-1,3-diol oder eine Isothiazolinon-Bromnitropropandiol-Zubereitung), Parfüm, Farbstoffe, Perlglanzmittel (beispielsweise Glykoldistearat) sowie Trübungsmittel oder auch Hautschutzmittel, wie sie beispielsweise in EP 522 506 beschrieben sind. Die Menge an derartigen Zusätzen liegt üblicherweise nicht über 12 Gew.% im Reinigungsmittel. Die Untergrenze des Einsatzes hängt von der Art des Zusatzstoffes ab und kann beispielsweise bei Farbstoffen bis zu 0,001 Gew.-% und darunter betragen. Vorzugsweise liegt die Menge an Hilfsstoffen zwischen 0,01 und 7 Gew.-%, insbesondere 0,1 und 4 Gew.-%.

Komplexbildner und Bleichmittel, wie etwa Natriumhypochlorit, müssen in dem erfindungsgemäßen Reinigungsmittel nicht enthalten sein, können jedoch gegebenenfalls in geringen Konzentrationen vorhanden sein.

Das Reinigungsmittel kann ein oder mehrere Treibmittel (INCI Propellants), üblicherweise in einer Menge von 1 bis 80 Gew.-%, vorzugsweise 1,5 bis 30 Gew.-%, insbesondere 2 bis 10 Gew.-%, besonders bevorzugt 2,5 bis 8 Gew.-%, äußerst bevorzugt 3 bis 6 Gew.-%, enthalten.

Treibmittel sind erfindungsgemäß üblicherweise Treibgase, insbesondere verflüssigte oder komprimierte Gase. Die Wahl richtet sich nach dem zu versprühenden Produkt und dem Einsatzgebiet.

Besonders bevorzugt enthält das Reinigungsmittel als ein oder mehrere Treibmittel Propan, Butan und/oder Isobutan, insbesondere Propan und Butan, äußerst bevorzugt Propan, Butan und Isobutan.

Noch ein weiterer Erfindungsgegenstand ist ein Verfahren zur Reinigung von harten Oberflächen im Haushalt, gegebenenfalls unter Benutzung eines erfindungsgemäßen (Mehrkammer-Systems) Erzeugnisses, bei dem die niedrig-viskosen Lösungen auf die zu reinigende Oberfläche aufgebracht und anschließend, gegebenenfalls nach einer angemessenen Einwirkzeit, mit klarem Wasser abgespült werden. Die hoch-viskose Flüssigkeit kann auch vor dem Abspülen mit einem Tuch, einem Schwamm, einer Bürste oder einem sonstigen zur Reinigung geeigneten Utensil auf Oberfläche verwischt oder verrieben werden, was z.B. bei Anwesenheit von Abrasivstoffen im Reinigungsmittel die Reinigungsleistung verbessert. Schließlich wird die Oberfläche gegebenenfalls mit einem trockenen Tuch abgetrocknet. Bei starker Verschmutzung der Oberfläche kann der Reinigungsvorgang anschließend wiederholt werden.

### Haarpflegemittel

Für die Verwendung als Körperpflegemittel, insbesondere als Haarpflegemittel können weitere Bestandteile, insbesondere ausgewählt aus milden Tensiden, Ölkörpern, Emulgatoren, Überfettungsmitteln, Perlglanzwachsen, Konsistenzgebern, Verdickungsmitteln, Polymeren, Siliconverbindungen, Fetten, Wachsen, Stabilisatoren, biogenen Wirkstoffen, Antischuppenmitteln, Filmbildnern, Quellmitteln, Antioxidantien, Hydrotropen, Konservierungsmitteln, Solubilisatoren, Parfümölen und Farbstoffen enthalten sein.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Vorbehandlungsmittel für Wäsche

Für die Verwendung als Vorbehandlungsmittel für Wäsche können neben dem hydrophob-modifiziertem Polymer und den oben genannten Tensiden weitere Komponenten in dem Wäschevorbehandlungsmittel enthalten sein, die dem Fachmann bekannt sind.

Es kann sich hierbei beispielsweise handeln um hydrophobe Komponenten, Gerüststoffe (Builder), Bleichmittel, Enzyme, Parfums/ Duftstoffe, Farbstoffe oder Emulsionshilfsmittel oder Kombinationen davon.

Als hydrophobe Komponenten eignen sich alle bekannten Öle, Fette und Wachse mineralischer, tierischer, pflanzlicher und synthetischer Herkunft. Bevorzugt sind Öl- und Fettkomponenten, vor allem flüssige Kohlenwasserstoffe mit 10 bis 32 C-Atomen, Fettsäureester mit insgesamt 12 bis 26 C-Atomen und beliebige Gemische davon. Geeignete Kohlenwasserstoffe sind vor allem Paraffine und Isoparaffine wie Isohexadecan oder n-Dodecan, aber auch andere, z.B. Triisobuten, Pentapropylen- oder 1,3-Di-(2-ethylhexyl)-cyclohexan.

Weitere Stoffe, die als hydrophobe Komponenten verwendet werden können, sind Dialkylether mit insgesamt 12 - 24 C-Atomen. Bevorzugt geeignete Dialkylether sind vor allem die aliphatischen Dialkylether mit jeweils 6 - 10 C-Atomen pro Alkylgruppe.

Darüber hinaus können alle anderen bekannten Ölkomponenten wie Vaseline, Pflanzenöle, synthetische Triglyceride wie z.B. Glyceryl-tricaprylat, aber auch Fette und Wachse sowie Silikonöle in den erfindungsgemäßen Mikroemulsionen enthalten sein.

In einer bevorzugten Ausführungsform der Erfindung werden hydrophobe Komponenten, vorzugsweise Paraffine, in Mengen von 5 bis 70 Gew.-%, besonders bevorzugt 8 bis 52 Gew.-%, jeweils bezogen auf die gesamte Mikroemulsion, eingesetzt.

Das Fleckenvorbehandlungsmittel kann Builder in Mengen, bezogen auf die Zusammensetzung, von 0 bis 30 Gew.-% enthalten.

Als Bleichmittel kommen alle üblicherweise in Wasch- und Reinigungsmitteln eingesetzten Bleichmittel in Betracht. Vorzugsweise wird jedoch Wasserstoffperoxid oder Phthalimidoperoxicapronsäure als Bleichmittel verwendet. Das Wäschevorbehandlungsmittel kann Bleichmittel in Mengen von 0 bis 30 Gew.-% enthalten.

Das erfindungsgemäße Fleckenvorbehandlungsmittel kann eines oder mehrere verschiedene amylolytische Enzyme, insbesondere α-Amylasen enthalten. Beispiele für kommerziell erhältliche Amylasen sind BAN^{®}, Termamyl^{®}, Purastar^{®}, Amylase-LT^{®}, Maxamyl^{®}, Duramyl^{®} und/oder Purafect^{®} OxAm.

Insbesondere an chemisch diversen Anschmutzungen kann es vorteilhaft sein, mehrere verschiedene wasch- und/oder reinigungsaktive Enzyme einzusetzen. Dazu gehören beispielsweise Proteasen, aber auch Lipasen, Cutinasen, Esterasen, Pullulanasen, Cellulasen, Hemicellulasen und/oder Xylanasen, sowie deren Gemische. Besonders bevorzugt sind Proteasen, Lipasen, β-Glucanasen und/oder Cellulasen. Weitere Enzyme erweitern die Reinigunsgleistung entsprechender Mittel um ihre jeweils spezifische enzymatische Leistung. Dazu gehören beispielsweise Oxidoreductasen oder Peroxidasen als Komponenten von enzymatischen Bleichsystemen, zum Beispiel Laccasen (WO 00/39306), β-Glucanasen (WO 99/06515 und WO 99/06516) oder Pektin-lösende Enzyme (WO 00/42145), die insbesondere in Spezialmitteln zum Einsatz kommen.

Das Fleckenvorbehandlungsmittel kann Enzyme in Mengen von 0 bis 3 Gew.-% enthalten.

Gegenstand der Erfindung ist in einer weiteren Ausführungsform die Verwendung der erfindungsgemäßen Textilvorbehandlungsmittel zur Textilvorbehandlung.

Das erfindungsgemäße Fleckenvorbehandlungsmittel ist besonders gut zur gezielten Vorbehandlung von Flecken auf verschiedenen Textilien geeignet. So können Kleidungsstücke und textile Gegenstände aus Vliesen, Filzen, Geweben oder Gewirken von Natur- und Chemiefasern wie Baumwolle, Wolle, Seide, Leinen, Kunstseiden, z. B. Viskose, Polyamid, Polyacryl, Polyester, Polyvinylchlorid, Elastan und allen weiteren üblicherweise eingesetzten Fasern sowie beliebigen Gemischen derselben an verschmutzten Stellen mit dem erfindungsgemäßen Fleckenvorbehandlungsmittel in Kontakt gebracht werden.

In einer Ausführungsform ist der Gegenstand der Erfindung ein Verfahren zur Entfernung von Flecken auf Textilien unter Verwendung eines erfindungsgemäßen Fleckenvorbehandlungsmittels.

Die Anwendung des Fleckenvorbehandlungsmittels erfolgt, indem es, vorzugsweise mit Hilfe einer der obengenannten Applikationsvorrichtungen, auf den Fleck aufgebracht wird und vorzugsweise 5 bis 15 Minuten einwirkt. Besonders bevorzugt wird das Fleckenvorbehandlungsmittel auf dem Fleck verrieben. Anschließend wird es entweder mit klarem Wasser ausgespült, oder aber das Textilstück wird einem sich anschließenden Textilwaschverfahren zugeführt.

## Patentansprüche

1. Mehrkomponenten-System, umfassend
a) eine erste wässrige Flüssigkeit, die mindestens ein hydrophob modifiziertes Polymer enthält,
b) eine zweite wässrige Flüssigkeit, die mindestens ein hydrophob modifiziertes Polymer und mindestens ein Tensid in einer Menge zwischen 0,05 und 20 Gew.-% enthält,
**dadurch gekennzeichnet, dass** es sich bei dem hydrophob modifizierten Polymer um ein hydrophob modifiziertes Polysaccharid handelt, das Verhältnis zwischen erster und zweiter Flüssigkeit zwischen 1:2 und 100:1 liegt und die Menge an hydrophob modifiziertem Polymer in den beiden Flüssigkeiten bis zu 5 Gew.-% beträgt.

2. Mehrkomponenten-System nach Anspruch 1, **dadurch gekennzeichnet, dass** der Modifizierungsgrad des hydrophob modifizierten Polymers zwischen 1 und 10 % liegt.

3. Mehrkomponenten-System nach Anspruch 1 oder 2, **dadurch gekennzeichnet, dass** das mindestens eine Tensid ausgewählt ist aus der Gruppe bestehend aus anionischen Tensiden, nichtionischen Tensiden, amphoteren Tensiden und zwitterionischen Tensiden.

4. Mehrkomponenten-System nach Anspruch 3, **dadurch gekennzeichnet, dass** das Tensid ausgewählt ist aus der Gruppe bestehend aus Alkylsulfaten, Alkylethersulfaten, Alkylsulfonaten, Alkylarylsulfonaten, Alkylbenzolsulfonaten, Alkylarylethersulfonaten, Alkyletheroxylaten, Fettalkoholethersulfaten, LAS, APG, Betainen und Fettalkoholethoxylaten.

5. Mehrkomponenten-System nach Anspruch 4, **dadurch gekennzeichnet, dass** es sich bei dem Tensid um Dodecylsulfat, Dodecylethersulfat und Fettalkoholethoxylate mit einem mittleren Ethoxylierungsgrad von weniger als 20 EO handelt.

6. Mehrkomponenten-System nach Anspruch 5, **dadurch gekennzeichnet, dass** es sich bei dem Tensid um Dodecylsulfat, Dodecylethersulfat und Fettalkoholethoxylate umfassend einen C₁₂-C₁₈-Febalkohol und 6-7 Ethoxylat-Einheiten handelt.

7. Mehrkomponenten-System nach Anspruch 6, **dadurch gekennzeichnet, dass** das Tensid in einer Menge zwischen 0 und 0,4 Ges.-% enthalten ist.

8. Mehrkomponenten-System nach einem der Ansprüche 1 bis 7, **dadurch gekennzeichnet, dass** es sich bei dem Mehrkomponenten-System um ein Zweikomponenten-System handelt und sich die zwei Flüssigkeiten in den beiden Kammern eines Doppelkammer-Systems befinden.

9. Mehrkomponenten-System nach Anspruch 8, **dadurch gekennzeichnet, dass** es sich bei dem Doppelkammer-System um eine Doppelkammerflasche oder ein Doppelkammerspray handelt.

10. Verfahren zur Herstellung einer hochviskosen wässrigen Flüssigkeit, **dadurch gekennzeichnet, dass** mindestens zwei Flüssigkeiten miteinander gemischt werden, wobei die erste der mindestens zwei Flüssigkeiten mindestens ein hydrophob modifiziertes Polymer enthält und die zweite der mindestens zwei Flüssigkeiten mindestens ein hydrophob modifiziertes Polymer und mindestens ein Tensid in einer Menge zwischen 0,05 und 20 Gew.-% enthält, **dadurch gekennzeichnet, dass** die Menge an hydrophob modifiziertem Polymer in den beiden Flüssigkeiten bis zu 5 Gew.% beträgt, dass die erste und zweite Flüssigkeit Im Verhältnis 1:2 bis 100:1 miteinander gemischt werden und dass es sich bei dem hydrophob modifizierten Polymer um ein hydrophob modifiziertes Polysaccharid handelt.

11. Verfahren nach Anspruch 10, **dadurch gekennzeichnet, dass** es sich bei dem Tensid um ein anionisches Tensid, nichtionisches Tensid, amphoteres Tensid oder zwitterionisches Tensid handelt.

12. Verfahren nach Anspruch 10 oder 11, **dadurch gekennzeichnet, dass** die Vermischung mit Hilfe eines Mehrkammersystems erfolgt.

13. Verwendung eines Mehrkomponenten-Systems nach einem der Ansprüche 1 bis 9 als kosmetisches Mittel.

14. Verwendung eines Mehrkomponenten-Systems nach einem der Ansprüche 1 bis 9 als Wasch- und/oder Spül- und/oder Reinigungsmittel.

## Claims

1. A multicomponent system comprising,
a) a first aqueous liquid which contains at least one hydrophobically modified polymer,
b) a second aqueous liquid which contains at least one hydrophobically modified polymer and at least one surfactant in an amount of between 0.05 and 20% by weight,
**characterised in that** the hydrophobically modified polymer is a hydrophobically modified polysaccharide, the ratio between the first and second liquid is between 1:2 and 100:1 and the amount of hydrophobically modified polymer in the two liquids is up to 5% by weight.

2. A multicomponent system according to claim 1, **characterised in that** the modification degree of the hydrophobically modified polymer is between 1 and 10%.

3. A multicomponent system according to claim 1 or claim 2, **characterised in that** at least one surfactant is selected from the group consisting of anionic surfactants, nonionic surfactants, amphoteric surfactants and zwitterionic surfactants.

4. A multicomponent system according to claim 3, **characterised in that** the surfactant is selected from the group consisting of alkyl sulfates, alkyl ether sulfates, alkyl sulfonates, alkylaryl sulfonates, alkylbenzene sulfonates, alkylaryl ether sulfonates, alkyl ether oxylates, fatty alcohol ether sulfates, LAS, APG, betaines and fatty alcohol ethoxylates.

5. A multicomponent system according to claim 4, **characterised in that** the surfactant is dodecyl sulfate, dodecyl ether sulfate and fatty alcohol ethoxylates with an average ethoxylation degree of less than 20 EO.

6. A multicomponent system according to claim 5, **characterised in that** the surfactant is dodecyl sulfate, dodecyl ether sulfate and fatty alcohol ethoxylates comprising a C₁₂-C₁₈ fatty alcohol and 6-7 ethoxylate units.

7. A multicomponent system according to claim 6, **characterised in that** the surfactant is contained in an amount of between 0 and 0.4% by weight.

8. A multicomponent system according to any of claims 1 to 7, **characterised in that** the multicomponent system is a two-component system and the two liquids are located in the two chambers of a double chamber system.

9. A multicomponent system according to claim 8, **characterised in that** the double chamber system is a double chamber bottle or a double chamber spray.

10. A method for producing a highly viscous aqueous liquid, **characterised in that** at least two liquids are mixed together wherein the first of said at least two liquids contains at least one hydrophobically modified polymer and the second of said at least two liquids contains at least one hydrophobically modified polymer and at least one surfactant in an amount of between 0.05 and 20% by weight, **characterised in that** the amount of hydrophobically modified polymer in the two liquids is up to 5% by weight, **in that** the first and the second liquids are mixed together in a ratio from 1:2 to 100:1 and **in that** the hydrophobically modified polymer is a hydrophobically modified polysaccharide.

11. A method according to claim 10, **characterised in that** the surfactant is an anionic surfactant, nonionic surfactant, amphoteric surfactant or zwitterionic surfactant.

12. A method according to claim 10 or claim 11, **characterised in that** mixing proceeds with the help of a multichamber system.

13. Use of a multicomponent system according to any of claims 1 to 9 as a cosmetic agent.

14. Use of a multicomponent system according to any of claims 1 to 9 as a washing and/or rinsing and/or cleaning agent.

## Revendications

1. Système à plusieurs composants comprenant
a) un premier liquide aqueux qui contient au moins un polymère modifié pour le rendre hydrophobe ;
b) un deuxième liquide aqueux qui contient au moins un polymère modifié pour le rendre hydrophobe et au moins un agent tensioactif en une quantité entre 0,05 et 20 % en poids ;
**caractérisé en ce que**, en ce qui concerne le polymère modifié pour le rendre hydrophobe, il s'agit d'un polysaccharide modifié pour le rendre hydrophobe, le rapport entre le premier et le deuxième liquide se situe entre 1:2 et 100:1 et la quantité du polymère modifié pour le rendre hydrophobe dans les deux liquides s'élève jusqu'à 5 % en poids.

2. Système à plusieurs composants selon la revendication 1, **caractérisé en ce que** le degré de modification du polymère modifié pour le rendre hydrophobe se situe entre 1 et 10 %.

3. Système à plusieurs composants selon la revendication 1 ou 2, **caractérisé en ce que** ledit au moins un agent tensioactif est choisi parmi le groupe constitué par des agents tensioactifs anioniques, des agents tensioactifs non ioniques, des agents tensioactifs amphotères et des agents tensioactifs zwitterioniques.

4. Système à plusieurs composants selon la revendication 3, **caractérisé en ce que** l'agent tensioactif est choisi parmi le groupe constitué par des alkylsulfates, des alkyléthersulfates, des alkylsulfonates, des alkylarylsulfonates, des alkylbenzènesulfonates, des alkylaryléthersulfonates, des alkylétheroxylates, des éthersulfates d'alcools gras, LAS, APG, des bétaïnes et des éthoxylates d'alcools gras.

5. Système à plusieurs composants selon la revendication 4, **caractérisé en ce que**, en ce qui concerne l'agent tensioactif, il s'agit du dodécylsulfate, du dodécyléthersulfate et d'éthoxylates d'alcools gras dont le degré d'éthoxylation moyen est inférieur à 20 OE.

6. Système à plusieurs composants selon la revendication 5, **caractérisé en ce que**, en ce qui concerne l'agent tensioactif, il s'agit du dodécylsulfate, du dodécyléthersulfate et d'éthoxylates d'alcools gras comprenant un alcool gras en C₁₂-C₁₈ et 6 à 7 unités d'éthoxylate.

7. Système à plusieurs composants selon la revendication 6, **caractérisé en ce que**, l'agent tensioactif est contenu en une quantité entre 0 et 0,4 % en poids.

8. Système à plusieurs composants selon l'une quelconque des revendications 1 à 7, **caractérisé en ce que**, en ce qui concerne le système à plusieurs composants, il s'agit d'un système à deux composants et les deux liquides se trouvent dans les deux chambres d'un système à chambre double.

9. Système à plusieurs composants selon la revendication 8, **caractérisé en ce que**, en ce qui concerne le système à chambre double, il s'agit d'un flacon à chambre double ou d'un spray à chambre double.

10. Procédé pour la préparation d'un liquide aqueux très visqueux, **caractérisé en ce qu'**on mélange l'un à l'autre au moins deux liquides, le premier desdits au moins deux liquides contenant au moins un polymère modifié pour le rendre hydrophobe et le deuxième desdits au moins deux liquides contenant au moins un polymère modifié pour le rendre hydrophobe et au moins un agent tensioactif en une quantité entre 0,05 et 20 % en poids, **caractérisé en ce que** la quantité du polymère modifié pour le rendre hydrophobe, dans les deux liquides, s'élève jusqu'à 5 % en poids, **en ce que** le premier et le deuxième liquide sont mélangés l'un à l'autre dans le rapport de 1:2 à 100:1, et **en ce que**, en ce qui concerne le polymère modifié pour le rendre hydrophobe, il s'agit d'un polysaccharide modifié pour le rendre hydrophobe.

11. Procédé selon la revendication 10, **caractérisé en ce que**, en ce qui concerne l'agent tensioactif, il s'agit d'un agent tensioactif anionique, d'un agent tensioactif non ionique, d'un agent tensioactif amphotère ou d'un agent tensioactif zwitterionique.

12. Procédé selon la revendication 10 ou 11, **caractérisé en ce que** le mélange a lieu à l'aide d'un système à plusieurs chambres.

13. Utilisation d'un système à plusieurs composants selon l'une quelconque des revendications 1 à 9, à titre d'agent cosmétique.

14. Utilisation d'un système à plusieurs composants selon l'une quelconque des revendications 1 à 9, à titre d'agent de lavage et/ou d'agent de rinçage et/ou d'agent de nettoyage.
